# EUROPEAN PATENT SPECIFICATION

(11) **EP 1 553 178 B1**
(45) Date of publication and mention of the grant of the patent: **05.03.2014**
(21) Application number: 03741222.8
(22) Date of filing: 07.07.2003
(51) Int. Cl.: C12N 15/09, C12N 7/01, C12N 9/12, A61K 48/00, A61P 35/00

(54) **Oncolytic virus growing selectively in tumor cells**
Onkolytisches Virus, das selektiv in Tumorzellen wächst
Virus oncolytique se propageant de manière sélective dans les cellules tumorales

(30) Priority: 08.07.2002 JP 2002198941
(43) Date of publication of application: 13.07.2005
(73) Proprietor: Oncolys BioPharma, Inc., Minato-ku Tokyo 105-0001 (JP)
(72) Inventor: SHIRAKIYA, Yoshiko, Kurashiki-shi, Okayama 710-0803 (JP); KAWASHIMA, Takeshi, Okayama-shi, Okayama 700-0914 (JP); KYO, Satoru, Ishikawa 921-8117 (JP); FUJIWARA, Toshiyoshi, Okayama-shi, Okayama 703-8281 (JP); TANAKA, Noriaki, Asakuchi-gun, Okayama 719-0252 (JP)
(74) Representative: Woods, Geoffrey Corlett
(86) International application number: PCT/JP2003/008573
(87) International publication number: WO 2004/005511

(56) References cited:
- WO-A-00/46355
- WO-A-01/73093
- DATABASE EPODOC EUROPEAN PATENT OFFICE, THE HAGUE, NL; CN1339584 13 March 2002 (2002-03-13), "Recombined virus for specific proliferation and high efficiency expression of anti-cancer gene in tumor cells and its constitution method" XP002361608 & CN 1 339 584 A (QIAN QIJUN) 13 March 2002 (2002-03-13) -& US 2005/048466 A1 (QIAN QIJUN ET AL) 3 March 2005 (2005-03-03)
- HIYAMA E ET AL: "Immunohistochemical detection of telomerase (hTERT) protein in human cancer tissues and a subset of cells in normal tissues." NEOPLASIA (NEW YORK, N.Y.) 2001 JAN-FEB, vol. 3, no. 1, January 2001 (2001-01), pages 17-26, XP002361553 ISSN: 1522-8002
- HOLT S E ET AL: "ROLE OF TELOMERASE IN CELLULAR PROLIFERATION AND CANCER" JOURNAL OF CELLULAR PHYSIOLOGY, LISS, NEW YORK, NY, US, vol. 180, no. 1, July 1999 (1999-07), pages 10-18, XP000965410 ISSN: 0021-9541
- ZHANG J. ET AL.: 'Identification of human uroplakin II promoter and its use in the construction of CG8840, a urothelium-specific adenovirus variant that eliminates established bladder tumors in combination with docetaxel' CANCER RES. vol. 62, no. 13, 01 July 2002, pages 3743 - 3750, XP002973938
- KIM J. ET AL.: 'Antitumoral effects of recombinant adenovirus YKL-1001, conditionally replicating in small alpha-fetoprotein-producing human liver cancer cells' CANCER LETTERS vol. 180, no. 1, 06 June 2002, pages 23 - 32, XP002973939
- KOMATA T. ET AL.: 'Caspase-8 gene therapy using the human telomerase reverse transcriptase promoter for malignant glioma cells' HUM. GENE THER. vol. 13, no. 9, 10 June 2002, pages 1015 - 1025, XP002973940
- KITAGAWA Y. ET AL.: 'Demethylating reagent 5-azacytidine inhibits telomerase activity in human prostate cancer cells through transcriptional repression of hTERT' CLIN. CANCER RES. vol. 6, no. 7, 2000, pages 2868 - 2875, XP002962899
- WIRTH T. ET AL.: 'A telomerase-dependent conditionally replicating adenovirus for selective treatment of cancer' CANCER RES. vol. 63, no. 12, 15 June 2003, pages 3181 - 3188, XP002973941

## Description

### TECHNICAL FIELD

The present invention relates to an adenovirus showing antitumor effect by replicating in tumor cells a polynucleotide contained in the virus and an anticancer agent comprising the virus.

### BACKGROUND ART

At present, gene therapy is performed as one method for treating cancers. However, since a gene is introduced into diseased tissue or the like with a non-replication competent virus vector in gene therapy, the gene can be applied to only those regions around target cells taking into consideration the safety of the human body. Also, in the gene therapy currently practiced, satisfactory therapeutic effect cannot be achieved because of low efficiency in gene transfer.

It is known that telomerase activity is often enhanced in malignantly transformed cells or immortalized cell strains, whereas telomerase activity is hardly detected in normal somatic cells excluding such as germ line cells, blood lineage cells and epithelial stem cells.

Under circumstances, it is a major object of the present invention to let a virus grow in tumor cells by utilizing the telomerase activated therein to thereby bring death to the tumor cells efficiently.

CN1339584A discloses an adenovirus, or adenoviral vector, that has the ability to infect and replicate selectivity in cells having telomerase activity. The virus or vector comprises the human telomerase catalytic subunit (hTERT) promoter or the telomerase RNA component gene (hTERC) promoter controlling expression of an immediate early gene.

WO 00/46355 discloses regulatory elements that promote transcription in cells that express telomerase reverse transcriptase (TERT). Oncolytic viruses are described, in which a toxin or a genetic element essential for viral replication is placed under control of the TERT promoter. As a result, the virus replicates preferentially in cells expressing TERT, and selectivity lyse cancer cells.

WO 01/73093 discloses replication-competent adenovirus vectors comprising co-transcribed first and second genes under transcriptional control of a heterologous, target cell-specific transcriptional regulatory element (TRE). The second gene is under translational control of an internal ribosome entry site. The vectors provide target cell-specific virus replication in applications such as cancer therapy and gene therapy.

Hiyama et al, Neoplasia 3(1), 17-26, 2001 examined hTERT expression at the cellular level in sectioned clinical materials using immunohistochemistry, and then compared these results to the same specimens in which the levels of telomerase activity had also been analyzed.

Holt and Shay, J. Cellular Physiology 180:10-18, 1999, review the role of telomerase in cellular proliferation and cancer. Zhang et al, Cancer Res. 62(13), 3743-3750, 2002 report the identification of human uroplakin II promoter and its use in the construction of a urothelium-specific adenovirus variant CG8840 that eliminates established bladder tumors in combination with docetaxel. Kim et al, Cancer Letters 180(1), 23-32, 2002 describes the antitumoral effects of a recombinant adenovirus YKL-1001 conditionally replicating in small alpha-fetoprotein-producing human lives cancer cells.

### BRIEF DESCRIPTION OF THE DRAWINGS

Fig. 1 shows a schematic drawing of the structure of a oncolytic virus repliacting selectively in tumor cells. A replication cassette consisting of hTERT promoter, E1A gene, IRES sequence and E1B gene is inserted in the E1 gene region which non-replication competent virus vectors lack.
Fig. 2 shows comparison of telomerase activities in human cancer cells and normal cells.
Fig. 3 shows the expression of E1A and E1B mRNAs and proteins after TRAD infection in human cancer cells and normal cells.
Fig. 4 shows the intracellular replication of the virus after TRAD infection in human cancer cells and normal cells.
Fig. 5 presents photographs showing, by staining with Coomassie brilliant blue, the cytotoxicity caused by TRAD in human cancer cells and normal cells.
Fig. 6 presents microscopic photographs showing the cytotoxicity caused by TRAD in human cancer cells and normal cells.
Fig. 7 presents graphs showing by means of XTT assay the cytotoxicity caused by TRAD in human cancer cells and normal cells.
Fig. 8 is a graph showing the antitumor effect produced by intratumoral, local administration of a non-replication competent, p53 gene-expressing adenovirus vector in an experiment using nude mice and human lung cancer cell H358.
Fig. 9 is a graph showing the antitumor effect produced by intratumoral, local administration of TRAD in an experiment using nude mice and human large bowel cancer cell SW620.

### DISCLOSURE OF THE INVENTION

The present inventors have found for the first time that, by infecting cancer cells with a virus having a telomerase promoter and replication ability, it is possible to let the virus replicate in the cancer cells and bring death to them. Thus, the present invention has been achieved.

Accordingly, the present invention provides a polynucleotide comprising an hTERT promoter, an adenovirus E1A gene, an IRES sequence and an adenovirus E1B gene in this order, wherein the hTERT promoter consists of the nucleotide sequence of SEQ ID NO: 4, the adenovirus E1A gene consists of the nucleotide sequence of SEQ ID NO: 1, the IRES sequence consists of the nucleotide sequence of SEQ ID NO: 3, and the adenovirus E1B gene consists of the nucleotide sequence of SEQ ID NO: 2.

The invention also provides:
- an adenovirus comprising the polynucleotide of the invention;
- a pharmaceutical composition comprising the adenovirus of the invention as active ingredient and a pharmaceutically acceptable carrier;
- an adenovirus of the invention for use in a method of treatment of the human or animal body by therapy;
- an adenovirus of the invention for use in a method of treating an hTERT-expressing cancer;
- a pharmaceutical composition of the invention for use in a method of treatment of the human or animal body by therapy; and
- a pharmaceutical composition of the invention for use in a method of treating an hTERT expressing cancer.

### DETAILED DESCRIPTION OF THE INVENTION

The present invention is characterized by bringing death to cancer cells by infecting cancer cells with a virus having a telomerase promoter and replication ability and letting the virus grow in the cancer cells, based on the finding that a wide variety of cancer cells have telomerase activity.

The virus used in the present invention is an adenovirus. Among adenovirus species, type 5 adenovirus is particularly preferable from the viewpoint of, for example, easiness in use.

Viruses have early (E) genes and late (L) genes involved in their DNA replication. E1 gene encodes a protein involved in the regulation of transcription of viral genome. It is known that E1 gene is composed of E1A, E1B and other elements. E1A protein encoded by E1A gene activates the transcription of a group of genes (E1B, E2, E4, etc.) necessary for the production of infectious virus. E1B protein encoded by E1B gene assists the accumulation of late gene (L gene) mRNA in the cytoplasm of the infected host cell to thereby inhibit the protein synthesis in the host cell. Thus, E1B protein promotes viral replication. The sequences of adenovirus E1A gene and E1B gene are shown in SEQ ID NO: 1 and SEQ ID NO: 2, respectively.

In the present invention, an E1 gene having an adenovirus E1A gene, an IRES sequence and an adenovirus E1B gene in this order (i.e., an E1 gene in which an IRES sequence is inserted between its E1A gene and E1B gene) is used. With the use of such an E1 gene, the replication ability of the virus of the invention will be high when a host cell has been infected with the virus.

As long as the effect of the invention can be achieved, at least one nucleotide may be inserted into at least one site selected from (a) between IRES sequence and E1A gene, (b) between IRES sequence and E1B gene, (c) upstream of E1A gene and (d) downstream of E1B gene. As long as the effect of the invention can be achieved, at least one, preferably several nucleotides may be substituted, deleted, inserted or added in the E1A gene, IRES sequence, E1B gene or E1 gene.

"IRES sequence" is a protein synthesis initiation signal specific to picornavirus. It is believed that this sequence serves as a ribosome-binding site because it contains a complementary sequence to the 3' terminal sequence of 18S ribosomal RNA. It is known that picornavirus-derived mRNA is translated via this sequence.

Translation efficiency from IRES sequence is high. Even from the middle of mRNA, protein synthesis is performed in a cap structure non-dependent manner. Therefore, in the virus of the present invention, both E1A gene and E1B gene located downstream of the IRES sequence are translated independently by a promoter from human telomerase. IRES sequence is shown in SEQ ID NO: 3.

In the present invention, the E1 gene has a promoter from human telomerase upstream thereof, because such a promoter is capable of promoting the replication of the virus of the invention in cancer cells having telomerase activity. The promoter is the hTERT promoter.

hTERT is a gene encoding human telomerase reverse transcriptase. A number of transcription factor-binding sequences are confirmed in a 1.4 kbp region upstream of the 5' end of this gene. This region is believed to be the hTERT promoter. In particular, a 181 bp sequence located upstream of the translation initiation site is a core region important for the expression of the downstream gene.

In the present invention, a sequence comprising this core region is used as the promoter from human telomerase. An upstream sequence of approximately 378 bp containing the core region completely is used. It has been confirmed that this sequence of approximately 378 bp is equivalent to the 181 bp core region alone in gene expression efficiency. The sequence of the hTERT promoter is shown in SEQ ID NO: 4.

A gene having the telomerase promoter of the invention and the E1 gene of the invention (a gene comprising E1A gene, IRES gene and E1B gene) may be obtained by conventional genetic engineering techniques.

As the E1 gene, an E1 gene from a known adenovirus having that gene may be used. Alternatively, E1A gene and E1B gene may be amplified from E1 gene-expressing cells (preferably, E1 gene-expressing 293 cells or the like) by RT-PCR and/or DNA-PCR using primers such as E1A-S, E1A-AS, E1B-S and E1B-AS. If necessary, their sequences are confirmed using a conventional method such as TA cloning. Then, E1A and E1B DNA fragments may be cut out using a known restriction enzyme such as EcoRI.

E1A and E1B may be inserted into a known vector such as pIRES by conventional genetic engineering techniques to thereby prepare E1A-IRES-E1B sequence within the vector. Subsequently, hTERT promoter sequence which was cut out with restriction enzymes such as MluI and BgIII may be inserted into the XhoI site or the like located upstream of E1A.

If necessary, cytomegalovirus (CMV) promoter contained in a known vector such as pShuttle may be removed with restriction enzymes such as MfeI and NheI; then, a sequence cut out from phTERT-E1A-IRES-E1B with restriction enzymes NheI and NotI may be inserted into the site (resultant vector is designated "pSh-hAIB").

From the resultant pSh-hAIB, a sequence comprising necessary portions (including hTERT promoter, E1A gene, IRES sequence and E1B gene) may be cut out with restriction enzymes such as I-CeuI and Pl-SceI, and then inserted into a viral DNA such as Adeno-X Viral DNA using a commercial kit such as Adeno-X Expression System (Clontech) (the resultant DNA is designated "AdenoX-hAIB").

The above-described sequence comprising hTERT promoter, E1A gene, IRES sequence and E1B gene may be inserted into any site of a viral gene as long as the effect of the present invention can be achieved. For example, in adenovirus for gene therapy from which E1 gene has been deleted, the above-described sequence is preferably inserted into the deleted site.

It is possible to linearize AdenoX-hAIB with a known restriction enzyme such as PacI and then transfect into cultured cells such as 293 cells, to thereby prepare a infectious recombinant adenovirus (the resultant virus is sometimes called the "virus of the present invention" or "TRAD"). The method of transfection is not particularly limited. From the viewpoint of efficiency, such methods as the calcium phosphate method or electroporation may be preferable.

The thus obtained virus of the present invention can be replicated by conventional methods for viral replication, e.g. infecting host cells such as 293 cells with the virus.

The virus of the present invention may be used as an anticancer agent. This anticancer agent may be used not only for treating cancers but also for preventing postoperative relapse of cancers, preventing cancer metastasis and/or for prophylaxis of cancers.

The kinds of cancers to which the anticancer agent of the invention is applied are not particularly limited. The anticancer agent is applicable to any kind of cancer. For example, the anticancer agent is effective for cancers in the stomach, large bowel, lung, liver, prostate, pancreas, esophagus, bladder, gallbladder/bile duct, breast, uterus, thyroid, ovary, etc. as well as brain tumor and osteosarcoma. Among all, the anticancer agent is especially effective for solid tumor.

The anticancer agent of the invention may be applied to diseased sites as it is. Alternatively, the anticancer agent may be introduced into humans (target cells or organs) by any known method, e.g. intravenous, intramuscular, intraperitoneal or subcutaneous injection; inhalation through the nasal cavity, oral cavity or lung; oral administration; administration in the form of suppository; and administration in the form of external medicine.

The virus of the invention may be treated, for example, by the lyophilization method to enable easy handling and then used alone, or prepared into pharmaceutical compositions by mixing with known pharmaceutically acceptable carriers such as excipients, fillers, binders, lubricants; or known additives (including such as buffers, isotonic agents, chelating agents, coloring agents, preservatives, flagrances, flavoring agents, and sweetening agents).

The anticancer agent of the present invention may be administered orally or parenterally depending on the form of the agent, e.g. oral administration agents such as tablets, capsules, powders, granules, pills, liquids, syrups, etc. and parenteral administration agents such as injections, external medicines, suppositories, eye drops, etc. Preferably, local injection into muscle or abdominal cavity, or intravenous injection may be enumerated.

Dose levels are selected appropriately depending on the kind of active ingredient, the administration route, the target of administration, and the age, body weight, sex, symptoms and other conditions of the patient. Usually, dose levels may be selected so that the virus of the invention (the active ingredient) is administered at a daily dose of about 10⁶-10¹¹ PFU, preferably about 10⁹-10¹¹ PFU. This amount may be administered once a day, or may be divided into several portions and administered at several times a day.

When the virus of the invention is administered, it is also possible to use a known immunosuppressant or the like to suppress the immunity of the living body to thereby make the viral infection easy.

Further, the virus of the invention may be used jointly with at least one anticancer agent selected from the group consisting of non-replication competent viruses (such as virus comprising p53 gene) used in conventional gene therapy, known anticancer agents and radiation.

The virus of the invention infected to the living body (cancer cells or cancer tissues) is capable of replicating in the cancer cells and bringing death to those cells. By thus bringing death to cancer cells, the virus of the invention can treat cancers, inhibit the growth of tumor cells, and prevent metastasis of cancer cells.

It is believed that there is an extremely low possibility that the anticancer agent of the invention will produce side effects for the reasons described below. Thus, the anticancer agent of the invention can be said a very safe preparation.
(1) There is little telomerase activity in normal somatic cells, and yet adenovirus itself is hard to be infected to suspending cells such as hematopoietic cells. Therefore, when adenovirus is used in the present invention, still higher selectivity for tumor kinds is obtained.
(2) Since the virus of the invention has replication ability, it is possible to use this virus at a lower concentration than that of conventional non-replication competent virus used in conventional gene therapy.
(3) Even when the virus of the invention has been administered in excess, antiviral action works through ordinary immune reaction in the living body.

### EXAMPLES

Hereinbelow, examples will be provided in order to illustrate the present invention in more detail. Needless to say, the present invention is not limited to these examples.

### Example 1

### <Preparation of TRAD>

An E1A gene of 899 bp was amplified from RNA extracted from 293 cells by RT-PCR using specific primers (E1A-S: SEQ ID NO: 5; E1A-AS: SEQ ID NO: 6). An E1B gene of 1823 bp was amplified from DNA extracted from 293 cells by DNA-PCR using primers (E1B-S: SEQ ID NO: 7; E1B-AS: SEQ ID NO: 8).

These PCR products were subjected to TA cloning (TA Cloning Kit Dual Promoter; Invitrogen) to thereby confirm their sequences. Then, DNA fragments of 899 bp (E1A) and 1823 bp (E1B) were cut out, respectively, with restriction enzyme EcoRI.

E1A and E1B were inserted into the MluI site and the SalI site of pIRES vector (Clontech), respectively, in the normal orientation (E1A-IRES-E1B).

A 455 bp hTERT promoter sequence which had been cut out with restriction enzymes MluI and BgIII was inserted into the XhoI site located upstream of the E1A of E1A-IRES-E1B (phTERT-E1A-IRES-E1B).

The cytomegalovirus (CMV) promoter contained in pShuttle vector was removed by treatment with restriction enzymes MfeI and NheI. Then, a 3828 bp sequence cut out from phTERT-E1A-IRES-E1B using restriction enzymes NheI and NotI was inserted into that site (pSh-hAIB).

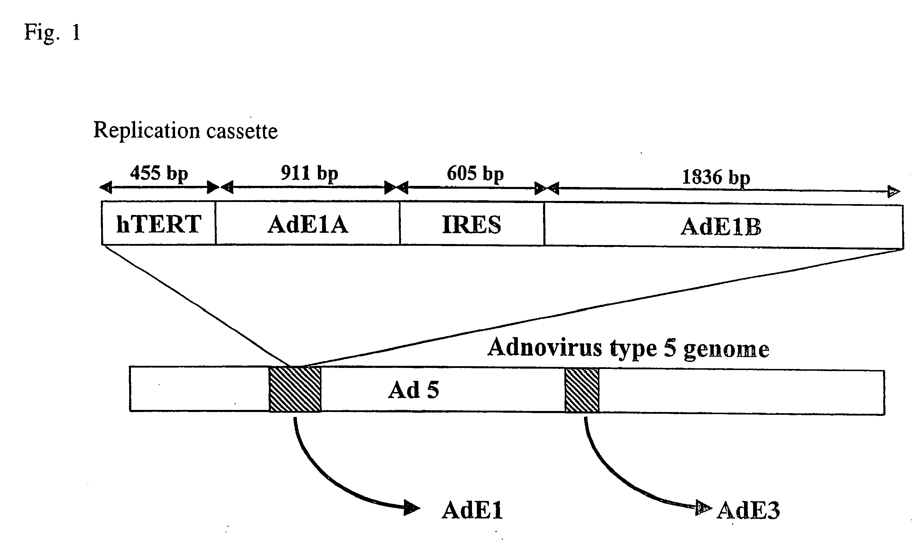

A 4381 bp sequence was cut out from pSh-hAIB using restriction enzymes I-CeuI and P1-SceI, and inserted into the Adeno-X Viral DNA of Adeno-X Expression System (Clontech) (AdenoX-hAIB). This AdenoX-hAIB was treated with restriction enzyme PacI for linearization and then transfected into 293 cells by the phosphate calcium method. Thus, a infectious recombinant adenovirus (TRAD) was prepared. A schematic drawing of TRAD is shown in Fig. 1.

### Example 2

### <Comparison of Telomerase Activities in Human Cancer Cells and Normal Cells>

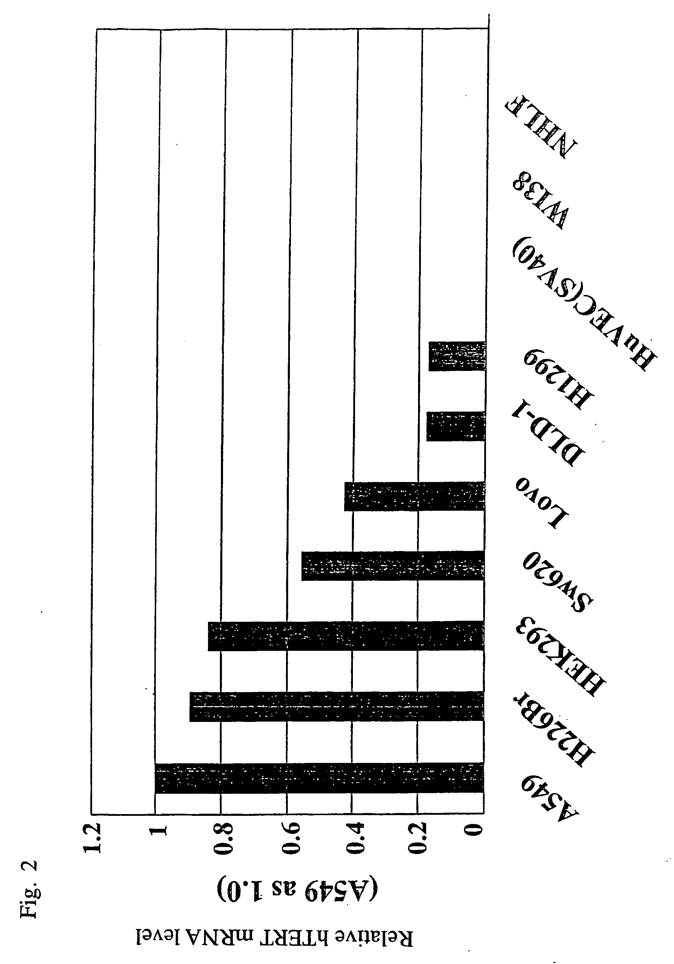

RNA was extracted from the following 10 kinds of cells using RNAzol (Cinna/Biotecx): human lung cancer cells (A549, H226Br and H1299); human large bowel cancer cells (SW620, DLD-1 and LoVo); human embryonic kidney cell 293; human umbilical vascular endothelial cell HUVEC immortalized by the introduction of SV40 gene; and human normal fibroblast cells (WI38 and NHLF). The resultant RNA was subjected to real time quantitative reverse transcription (RT)-PCR using Light Cycler DNA TeloTAGGG Kit (Roche Molecular Biochemicals), followed by comparison of expression levels of hTERT gene in respective cells. The results are shown in Fig. 2.

When expression levels were compared taking the level in A549 cells (which showed the highest expression) as 1.0, hTERT gene expression from 0.18 to 1.00 was observed in cancer cells (such as A549, H226Br, H1299, SW620, DLD-1, Lovo) and 293 cells, whereas no expression was detected in immortalized cell HuVEC and normal cells (such as WI38, NHLF).

### Example 3

### <Expression of E1A and E1B mRNAs and Proteins after TRAD Infection in Human Cancer Cells and Normal Cells>

Human large bowel cancer cell SW620 and human normal fibroblast cell WI38 were cultured *in vitro*. Then, each cell was infected with TRAD at concentrations of MOI (multiplicity of infection) 0.1 and 1, followed by recovery of RNA after 36 hours. As a positive control, 293 cells were used.

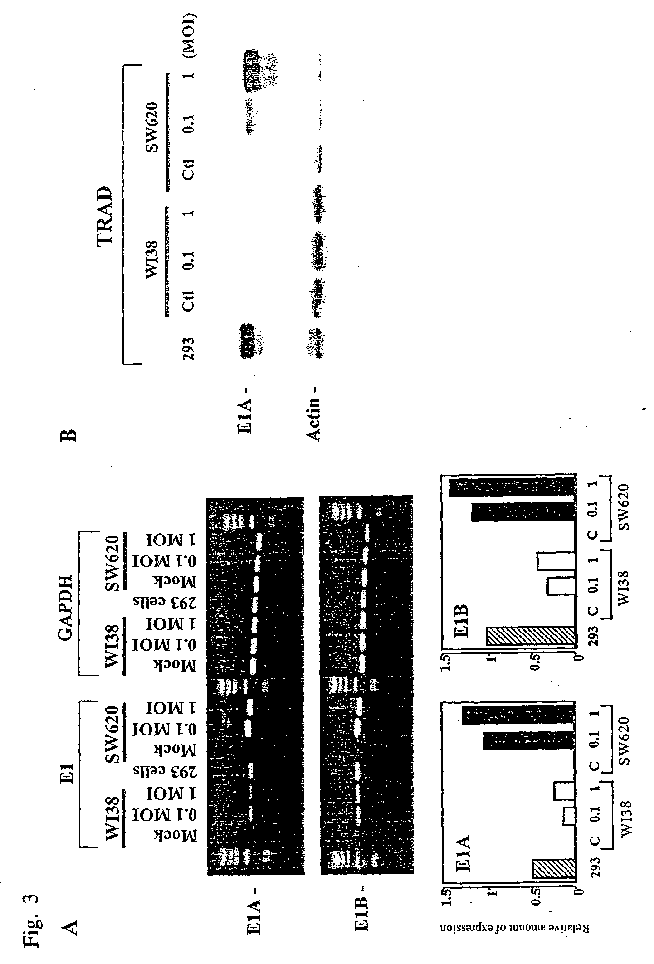

The recovered RNA was reverse-transcribed using GeneAmp RNA PCR Core Kit. The resultant DNA was amplified 30 cycles in GeneAmp PCR System 9700 Thermal Cycler (PE Applied Biosystems) using primers for E1A gene and E1B gene. The PCR products were electrophoresed on 1.2% agarose gel and stained with ethidium bromide to thereby visualize bands (upper two panels in Fig. 3A). The intensities of the bands were measured with an image analyzer, quantitatively determined using GAPDH as an internal control and then shown in graphs (the bottom panel in Fig. 3A).

Human large bowel cancer cell SW620 and human normal fibroblast cell WI38 were cultured *in vitro.* Then, each cell was infected with TRAD at concentrations of MOI 0.1 and 1. After 48 hours, adherent cells were recovered and reacted in a lysis solution for 30 minutes, followed by centrifugation. The protein concentration in the resultant supernatant was measured. Briefly, the supernatant was electrophoresed on 12% polyacrylamide gel and transferred onto a membrane. Then, Western blot analysis was performed with anti-adenovirus 5 E1A antibody (PharMingen International). The results are shown in Fig. 3B.

While strong expression of E1A gene (502 bp) and E1B gene (543 bp) was clearly observed as a result of TRAD infection in cancer cell SW620, only weak expression of these genes was observed in normal cell WI38 (Fig. 3A). In the positive control 293 cells, medium expression of these genes was observed.

The results of Western blot analysis revealed that expression of E1A protein increased in SW620 as the concentration of TRAD increased from MOI 0.1 to 1 (Fig. 3B). On the other hand, expression of E1A protein was detected little in WI38 even when TRAD was used at MOI 1.

### Example 4

### <Examination of Intracellular Viral Replication after TRAD Infection in Human Cancer Cells and Normal Cells >

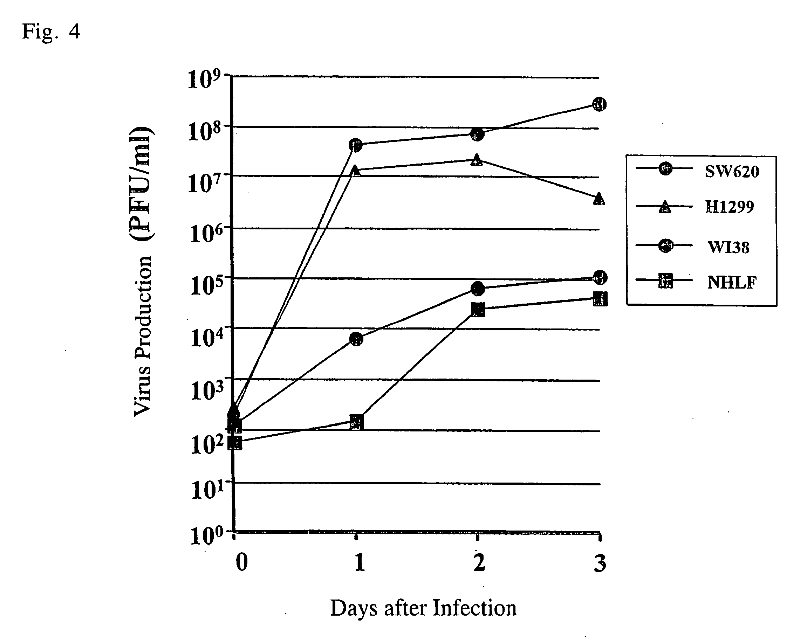

Human cancer cells (SW620 and H1299) and human normal cells (WI38 and NHLF) were infected with TRAD at MOI 1 for 2 hours at 37 °C. Then, the TRAD-containing culture broth was discarded. After cells were washed with a fresh culture broth once, a fresh culture broth was added further. Immediately thereafter (*i*.*e*., on day 0), cells were recovered with a scraper and subjected to repetition of freezing and thawing. Then, they were suspended in 1 ml of a culture broth. Further, virus was recovered on day 1, 2, 3, 5 and 7 in the same manner, followed by measurement of viral titer. The results are shown in Fig. 4.

In normal cells WI38 and NHLF, TRAD increased from 10² PFU on day 1 to about 10⁵ PFU on day 3 showing 100- to 1000-fold growth. On the other hand, in cancer cells SW620 and H1299, TRAD increased to 10⁷-10⁸ PFU showing 10⁵- to 10⁶-fold growth. Thus, viral growth specific to cancer cells was confirmed.

### Example 5

### <Cytotoxic Activity of TRAD in Human Cancer Cells and Normal Cells>

Five kinds of human cancer cells (SW620, H1299, A549, DLD-1 and H226Br) were plated on 24-well plates at 6-8 x 10⁴ cells/well, and two kinds of human normal cells (WI38 and NHLF) were plated on 24-well plates at 2-4 x 10⁴ cells/well. After 24 hours, they were infected with TRAD at MOI 0.01, 0.1, 1, 2 and 5. Niney-six hours after the infection, morphological changes in SW620, DLD-1 and NHLF cells were observed under microscopy. Further, culture broth was discarded from all of the cells. Then, viable cells were stained with Coomassie brilliant blue, and macroscopic images were taken into with a scanner.

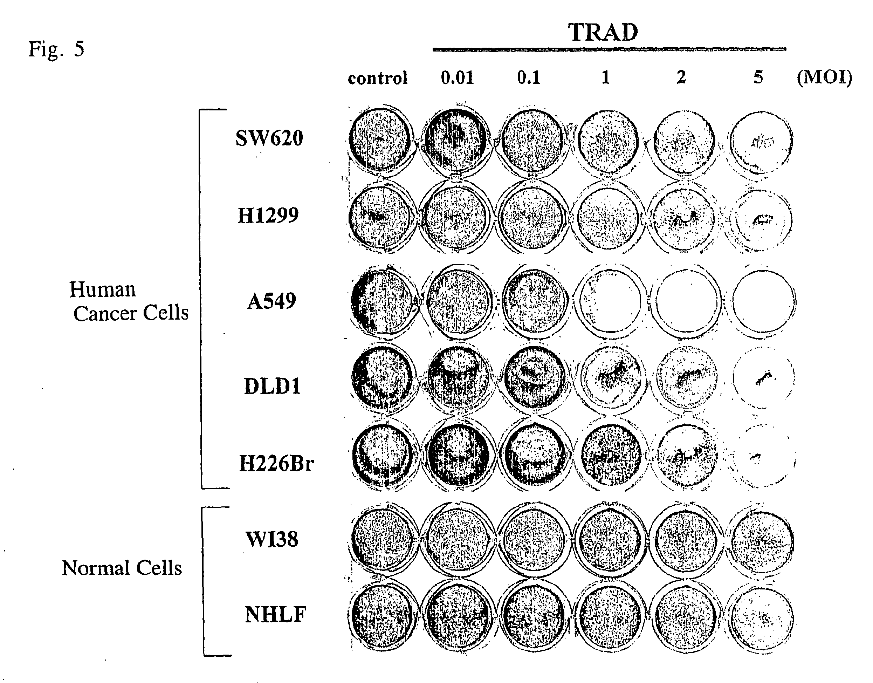

SW620 and H1299 were plated at 10⁴ cells/well and NHLF was plated at 5 x 10³ cells/well, respectively, on 96-well plates. Cells were infected with TRAD at MOI 0 (non-infected cells), 0.01, 0.1 and 1. Then, the numbers of viable cells were measured by XTT assay on day 1, 2, 3, 5 and 7. The viable cell count was determined for each four wells. Taking the count in the non-infected cells as 1.0, counts in other cells were represented in graphs in means +/- SDs. Respective results are shown in Figs. 5, 6 and 7.

In cancer cells SW620, H1299, A549, DLD-1 and H226Br, cell counts decrease and areas stained with blue reduce in a TRAD concentration-dependant manner. On the other hand, in normal cells WI38 and NHLF, no remarkable decrease in the number of viable cells stained with blue was recognized (Fig. 5).

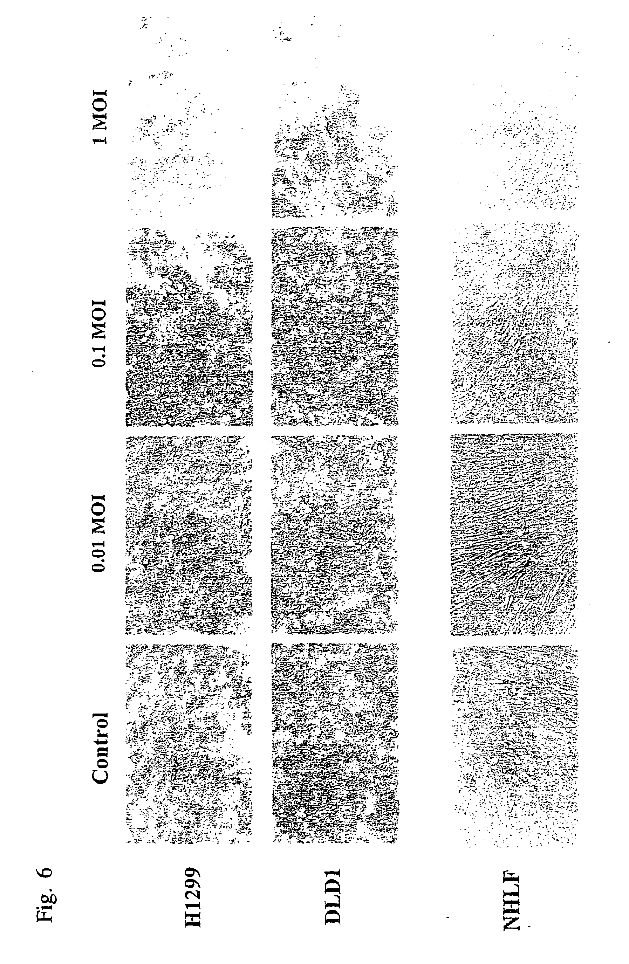

In the microscopic observation, SW620 and DLD-1 cells were peeled off from the plate bottom, became round-shaped and showed decrease in cell density; on the other hand, NHLF cells showed little morphological change and no decrease in cell count (Fig. 6).

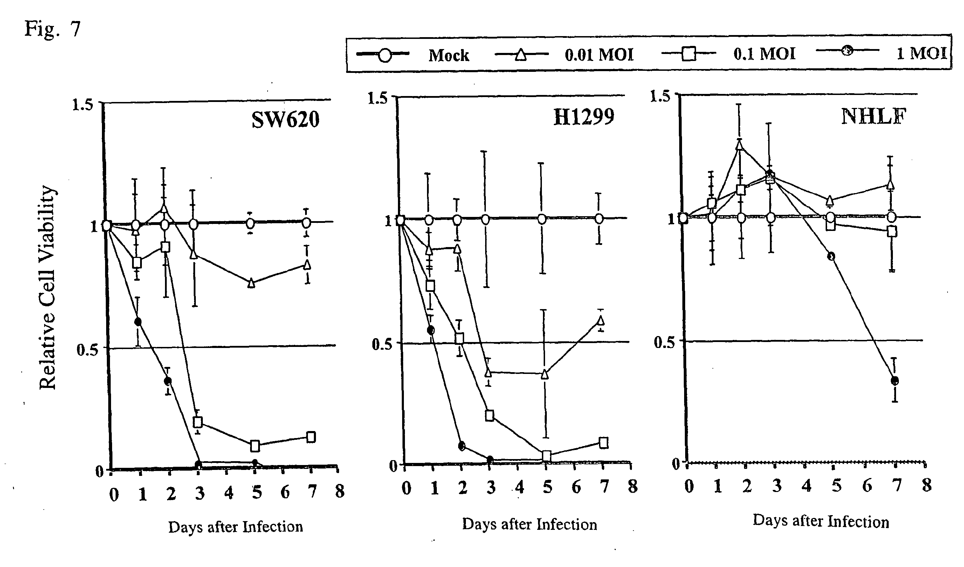

In SW620 and H1299 cells, almost 100% cell death was observed by day 3 as a result of TRAD infection at MOI 1. More than 80% decrease in cell count was recognized even at MOI 0.1. On the other hand, NHLF showed almost no decrease in cell count even on day 3. Although NHLF showed about 60% decrease in cell count on day 7 when TRAD was used at MOI 1, it indicated no viral influence at MOI 0.01 (Fig. 7).

### Example 6

### <Examination of the Antitumor Activity of TRAD in Animal Models>

Human lung cancer cell H358 was transplanted subcutaneously into the back of 5-6 week-old nude mice at 5 x 10⁶ cells/mouse. When the tumor became approximately 5-6 mm in diameter, a non-replication competent adenovirus vector (Ad-p53) was injected intratumorally and locally for consecutive two days at 1 x 10⁸ PFU, 3 x 10⁸ PFU and 1 x 10⁹ PFU per day. Then, two axes of each tumor crossing at right angles were measured at regular intervals. The estimated tumor weight was calculated by the following formula: (major axis) x (minor axis)²/2. As a control, a non-replication competent adenovirus vector dl312 containing no inserted gene was used.

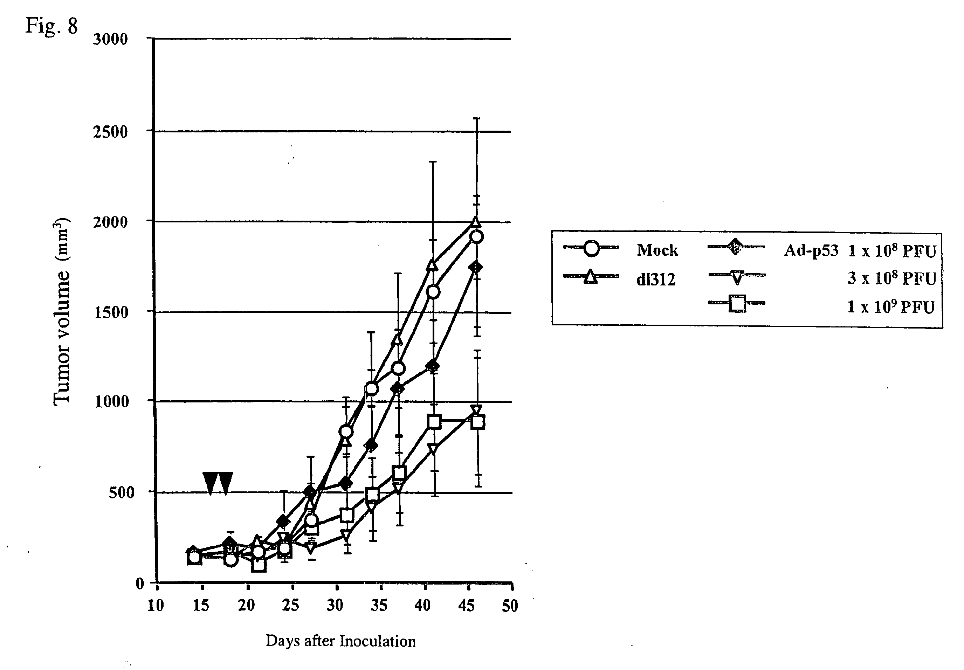

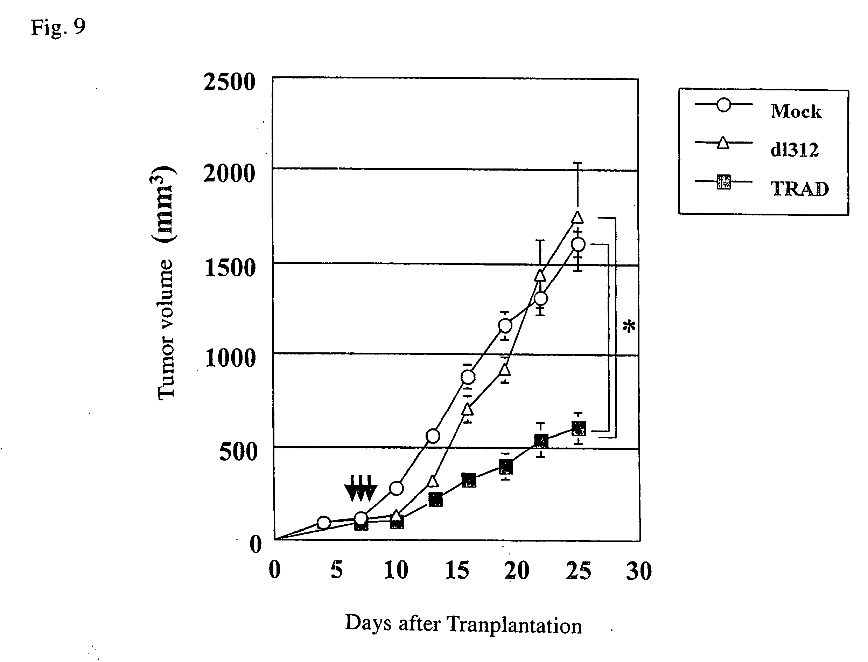

Human large bowel cancer cell SW620 was transplanted subcutaneously into the back of 5-6 week-old nude mice at 5 x 10⁶ cells/mouse. When the tumor became approximately 5-6 mm in diameter, 2 x 10⁷ PFU of d131/day and 4 x 10³ PFU of TRAD/day were injected intratumorally and locally for consecutive three days. The axes of each tumor were measured in the same manner as described above, followed by calculation of the estimated tumor weight. The results are shown in Figs. 8 and 9 (the term "Mock" appearing in these Figures represents control to which PBS (phosphate buffered saline) was administered).

Administration of Ad-p53 at 3 x 10⁸ PFU and 1 x 10⁹ PFU inhibited the growth of H358 tumor significantly (p<0.05). However, administration of Ad-p53 at 1 x 10⁸ PFU revealed no significant growth inhibition (Fig. 8). Administration of dl312 (control) indicated no influence upon tumor growth.

Intratumoral administration of TRAD at 4 x 10³ PFU, which is extremely lower than the concentration of Ad-p53 that showed antitumor effect, inhibited the growth of SW620 tumor significantly (p<0.05). Administration of dl312 (control) indicated no influence upon tumor growth.

From what have been described above, it is understood that the virus of the present invention grows efficiently in cancer cells and brings death to them. Further, since the virus of the invention has the ability to grow, it is capable of manifesting potent anti-cancer effect even at a low concentration. Thus, it is also possible to reduce side effect by administering the virus at a low concentration.

## Claims

1. A polynucleotide comprising an hTERT promoter, an adenovirus E1A gene, an IRES sequence and an adenovirus E1B gene in this order, wherein the hTERT promoter consists of the nucleotide sequence of SEQ ID NO: 4, the adenovirus E1A gene consists of the nucleotide sequence of SEQ ID NO: 1, the IRES sequence consists of the nucleotide sequence of SEQ ID NO: 3, and the adenovirus E1B gene consists of the nucleotide sequence of SEQ ID NO: 2.

2. An adenovirus comprising the polynucleotide according to claim 1.

3. A pharmaceutical composition comprising the adenovirus according to claim 2 as an active ingredient and a pharmaceutically acceptable carrier.

4. An adenovirus as defined in claim 2 for use in a method of treatment of the human or animal body by therapy.

5. An adenovirus as defined in claim 2 for use in a method of treating an hTERT-expressing cancer.

6. The adenovirus for use according to claim 5, wherein the hTERT-expressing cancer is at least one cancer selected from the group consisting of stomach cancer, large bowel cancer, lung cancer, liver cancer, prostate cancer, pancreas cancer, esophagus cancer, bladder cancer, gallbladder/bile duct cancer, breast cancer, uterine cancer, thyroid cancer and ovarian cancer.

7. The adenovirus for use according to claim 5, wherein the hTERT-expressing cancer is at least one selected from the group consisting of osteosarcoma and brain tumor.

8. A pharmaceutical composition as defined in claim 3 for use in a method of treatment of the human or animal body by therapy.

9. A pharmaceutical composition as defined in claim 3 for use in a method of treating an hTERT-expressing cancer.

10. The pharmaceutical composition for use according to claim 9, wherein the hTERT-expressing cancer is at least one cancer selected from the group consisting of stomach cancer, large bowel cancer, lung cancer, liver cancer, prostate cancer, pancreas cancer, esophagus cancer, bladder cancer, gallbladder/bile duct cancer, breast cancer, uterine cancer, thyroid cancer and ovarian cancer.

11. The pharmaceutical composition for use according to claim 9, wherein the hTERT-expressing cancer is at least one selected from the group consisting of osteosarcoma and brain tumor.

## Patentansprüche

1. Polynucleotid, umfassend einen hTERT-Promotor, ein Adenovirus-E1A-Gen, eine IRES-Sequenz und ein Adenovirus-E1B-Gen in dieser Reihenfolge, wobei der hTERT-Promotor aus der Nucleotidsequenz von SEQ ID NO: 4 besteht, das Adenovirus-E1A-Gen aus der Nucleotidsequenz von SEQ ID NO: 1 besteht, die IRES-SEQUENZ aus der Nucleotidsequenz von SEQ ID NO: 3 besteht und das Adenovirus-E1B-Gen aus der Nucleotidsequenz von SEQ ID NO: 2 besteht.

2. Adenovirus, das das Polynucleotid gemäß Anspruch 1 umfasst.

3. Pharmazeutische Zusammensetzung, die das Adenovirus gemäß Anspruch 2 als aktives Ingrediens und einen pharmazeutisch annehmbaren Träger umfasst.

4. Adenovirus, wie in Anspruch 2 definiert, zur Verwendung in einem Verfahren zur Behandlung des Menschen- oder Tierkörpers durch Therapie.

5. Adenovirus, wie in Anspruch 2 definiert, zur Verwendung in einem Verfahren zur Behandlung eines hTERT-exprimierenden Krebses.

6. Adenovirus zur Verwendung gemäß Anspruch 5, wobei der hTERT-exprimierende Krebs wenigstens ein Krebs, ausgewählt aus der Gruppe, bestehend aus Magenkrebs, Dickdarmkrebs, Lungenkrebs, Leberkrebs, Prostatakrebs, Pankreaskrebs, Ösophaguskrebs, Blasenkrebs, Gallenblasen/Gallenleiter-Krebs, Brustkrebs, Uteruskrebs, Schilddrüsenkrebs und Eierstockkrebs, ist.

7. Adenovirus zur Verwendung gemäß Anspruch 5, wobei der hTERT-exprimierende Krebs wenigstens einer, ausgewählt aus der Gruppe, bestehend aus Osteosarkom und Gehirntumor, ist.

8. Pharmazeutische Zusammensetzung, wie in Anspruch 3 definiert, zur Verwendung in einem Verfahren zur Behandlung des Menschen- oder Tierkörpers durch Therapie.

9. Pharmazeutische Zusammensetzung, wie in Anspruch 3 definiert, zur Verwendung in einem Verfahren zur Behandlung eines hTERT-exprimierenden Krebses.

10. Pharmazeutische Zusammensetzung zur Verwendung gemäß Anspruch 9, wobei der hTERT-exprimierende Krebs wenigstens ein Krebs, ausgewählt aus der Gruppe, bestehend aus Magenkrebs, Dickdarmkrebs, Lungenkrebs, Leberkrebs, Prostatakrebs, Pankreaskrebs, Ösophaguskrebs, Blasenkrebs, Gallenblasen/Gallenleiter-Krebs, Brustkrebs, Uteruskrebs, Schilddrüsenkrebs und Eierstockkrebs, ist.

11. Pharmazeutische Zusammensetzung zur Verwendung gemäß Anspruch 9, wobei der hTERT-exprimierende Krebs wenigstens einer, ausgewählt aus der Gruppe, bestehend aus Osteosarkom und Gehirntumor, ist.

## Revendications

1. Polynucléotide comprenant, dans l'ordre indiqué, un promoteur hTERT, un gène E1A d'adénovirus, une séquence IRES et un gène E1B d'adénovirus, dans lequel le promoteur hTERT consiste en la séquence de nucléotides donnée en tant que Séquence N° 4, le gène E1A d'adénovirus consiste en la séquence de nucléotides donnée en tant que Séquence N° 1, la séquence IRES consiste en la séquence de nucléotides donnée en tant que Séquence N° 3, et le gène E1B d'adénovirus consiste en la séquence de nucléotides donnée en tant que Séquence N° 2.

2. Adénovirus comprenant un polynucléotide conforme à la revendication 1.

3. Composition pharmaceutique comprenant un adénovirus conforme à la revendication 2, en tant qu'ingrédient actif, et un véhicule pharmacologiquement admissible.

4. Adénovirus conforme à la revendication 2 pour utilisation dans un procédé de traitement thérapeutique du corps d'un humain ou d'un animal.

5. Adénovirus conforme à la revendication 2 pour utilisation dans un procédé de traitement d'un cancer où le gène hTERT est exprimé.

6. Adénovirus pour utilisation conforme à la revendication 5, pour lequel le cancer où le gène hTERT est exprimé est un cancer, au nombre d'au moins un, choisi dans l'ensemble formé par les suivants : cancer de l'estomac, cancer du gros intestin, cancer du poumon, cancer du foie, cancer de la prostate, cancer du pancréas, cancer de l'oesophage, cancer de la vessie, cancer de la vésicule et des voies biliaires, cancer du sein, cancer de l'utérus, cancer de la thyroïde, et cancer des ovaires.

7. Adénovirus pour utilisation conforme à la revendication 5, pour lequel le cancer où le gène hTERT est exprimé est un cancer, au nombre d'au moins un, choisi dans l'ensemble formé par un ostéosarcome et une tumeur cérébrale.

8. Composition pharmaceutique conforme à la revendication 3 pour utilisation dans un procédé de traitement thérapeutique du corps d'un humain ou d'un animal.

9. Composition pharmaceutique conforme à la revendication 3 pour utilisation dans un procédé de traitement d'un cancer où le gène hTERT est exprimé.

10. Composition pharmaceutique pour utilisation conforme à la revendication 9, pour lequel le cancer où le gène hTERT est exprimé est un cancer, au nombre d'au moins un, choisi dans l'ensemble formé par les suivants : cancer de l'estomac, cancer du gros intestin, cancer du poumon, cancer du foie, cancer de la prostate, cancer du pancréas, cancer de l'oesophage, cancer de la vessie, cancer de la vésicule et des voies biliaires, cancer du sein, cancer de l'utérus, cancer de la thyroïde, et cancer des ovaires.

11. Composition pharmaceutique pour utilisation conforme à la revendication 9, pour lequel le cancer où le gène hTERT est exprimé est un cancer, au nombre d'au moins un, choisi dans l'ensemble formé par un ostéosarcome et une tumeur cérébrale.


### SEQUENCE LISTING

<110> KANSAI Technology Licensing Organization, Toshiyoshi FUJIWARA, Noriaki TANAKA, Satoru KY0
<120> Oncolyis with Tumor-specific Replication-competent Virus
<130> P03-75
<150> JP2002-198941
   <151> 2002-07-08
<160> 8
<170> PatentIn Ver. 2.1
<210> 1
   <211> 899
   <212> DNA
   <213> 293 cell
<400> 1
   acaccgggac tgaaaatgag acatattatc tgccacggag gtgttattac cgaagaaatg      60
   gccgccagtc ttttggacca gctgatcgaa gaggtactgg ctgataatct tccacctcct     120
   agccattttg aaccacctac ccttcacgaa ctgtatgatt tagacgtgac ggcccccgaa     180
   gatcccaacg aggaggcggt ttcgcagatt tttcccgact ctgtaatgtt ggcggtgcag     240
   gaagggattg acttactcac ttttccgccg gcgcccggtt ctccggagcc gcctcacctt     300
   tcccggcagc ccgagcagcc ggagcagaga gccttgggtc cggtttctat gccaaacctt     360
   gtaccggagg tgatcgatct tacctgccac gaggctggct ttccacccag tgacgacgag     420
   gatgaagagg gtgaggagtt tgtgttagat tatgtggagc accccgggca cggttgcagg     480
   tcttgtcatt atcaccggag gaatacgggg gacccagata ttatgtgttc gctttgctat     540
   atgaggacct gtggcatgtt tgtctacagt cctgtgtctg aacctgagcc tgagcccgag     600
   ccagaaccgg agcctgcaag acctacccgc cgtcctaaaa tggcgcctgc tatcctgaga     660
   cgcccgacat cacctgtgtc tagagaatgc aatagtagta cggatagctg tgactccggt     720
   ccttctaaca cacctcctga gatacacccg gtggtcccgc tgtgccccat taaaccagtt     780
   gccgtgagag ttggtgggcg tcgccaggct gtggaatgta tcgaggactt gcttaacgag     840
   cctgggcaac ctttggactt gagctgtaaa cgccccaggc cataaggtgt aaacctgtg      899
<210> 2
   <211> 1823
   <212> DNA
   <213> 293 cell
<400> 2
   ctgacctcat ggaggcttgg gagtgtttgg aagatttttc tgctgtgcgt aacttgctgg 60
   aacagagctc taacagtacc tcttggtttt ggaggtttct gtggggctca tcccaggcaa 120
   agttagtctg cagaattaag gaggattaca agtgggaatt tgaagagctt ttgaaatcct 180
   gtggtgagct gtttgattct ttgaatctgg gtcaccaggc gcttttccaa gagaaggtca 240
   tcaagacttt ggatttttcc acaccggggc gcgctgcggc tgctgttgct tttttgagtt 300
   ttataaagga taaatggagc gaagaaaccc atctgagcgg ggggtacctg ctggattttc 360
   tggccatgca tctgtggaga gcggttgtga gacacaagaa tcgcctgcta ctgttgtctt 420
   ccgtccgccc ggcgataata ccgacggagg agcagcagca gcagcaggag gaagccaggc 480
   ggcggcggca ggagcagagc ccatggaacc cgagagccgg cctggaccct cgggaatgaa 540
   tgttgtacag gtggctgaac tgtatccaga actgagacgc attttgacaa ttacagagga 600
   tgggcagggg ctaaaggggg taaagaggga gcggggggct tgtgaggcta cagaggaggc 660
   taggaatcta gcttttagct taatgaccag acaccgtcct gagtgtatta cttttcaaca 720
   gatcaaggat aattgcgcta atgagcttga tctgctggcg cagaagtatt ccatagagca 780
   gctgaccact tactggctgc agccagggga tgattttgag gaggctatta gggtatatgc 840
   aaaggtggca cttaggccag attgcaagta caagatcagc aaacttgtaa atatcaggaa 900
   ttgttgctac atttctggga acggggccga ggtggagata gatacggagg atagggtggc 960
   ctttagatgt agcatgataa atatgtggcc gggggtgctt ggcatggacg gggtggttat 1020
   tatgaatgta aggtttactg gccccaattt tagcggtacg gttttcctgg ccaataccaa 1080
   ccttatccta cacggtgtaa gcttctatgg gtttaacaat acctgtgtgg aagcctggac 1140
   cgatgtaagg gttcggggct gtgcctttta ctgctgctgg aagggggtgg tgtgtcgccc 1200
   caaaagcagg gcttcaatta agaaatgcct ctttgaaagg tgtaccttgg gtatcctgtc 1260
   tgagggtaac tccagggtgc gccacaatgt ggcctccgac tgtggttgct tcatgctagt 1320
   gaaaagcgtg gctgtgatta agcataacat ggtatgtggc aactgcgagg acagggcctc 1380
   tcagatgctg acctgctcgg acggcaactg tcacctgctg aagaccattc acgtagccag 1440
   ccactctcgc aaggcctggc cagtgtttga gcataacata ctgacccgct gttccttgca 1500
   tttgggtaac aggagggggg tgttcctacc ttaccaatgc aatttgagtc acactaagat 1560
   attgcttgag cccgagagca tgtccaaggt gaacctgaac ggggtgtttg acatgaccat 1620
   gaagatctgg aaggtgctga ggtacgatga gacccgcacc aggtgcagac cctgcgagtg 1680
   tggcggtaaa catattagga accagcctgt gatgctggat gtgaccgagg agctgaggcc 1740
   cgatcacttg gtgctggcct gcacccgcgc tgagtttggc tctagcgatg aagatacaga 1800
   ttgaggtact gaaatgtgtg ggc 1823
<210> 3
   <211> 605
   <212> DNA
   <213> 293 cell
<400> 3
   tgcatctagg gcggccaatt ccgcccctct ccctcccccc cccctaacgt tactggccga 60
   agccgcttgg aataaggccg gtgtgcgttt gtctatatgt gattttccac catattgccg 120
   tcttttggca atgtgagggc ccggaaacct ggccctgtct tcttgacgag cattcctagg 180
   ggtctttccc ctctcgccaa aggaatgcaa ggtctgttga atgtcgtgaa ggaagcagtt 240
   cctctggaag cttcttgaag acaaacaacg tctgtagcga ccctttgcag gcagcggaac 300
   cccccacctg gcgacaggtg cctctgcggc caaaagccac gtgtataaga tacacctgca 360
   aaggcggcac aaccccagtg ccacgttgtg agttggatag ttgtggaaag agtcaaatgg 420
   ctctcctcaa gcgtattcaa caaggggctg aaggatgccc agaaggtacc ccattgtatg 480
   ggatctgatc tggggcctcg gtgcacatgc tttacatgtg tttagtcgag gttaaaaaaa 540
   cgtctaggcc ccccgaacca cggggacgtg gttttccttt gaaaaacacg atgataagct 600
   tgcca 605
<210> 4
   <211> 455
   <212> DNA
   <213> 293 cell
<400> 4
   tggcccctcc ctcgggttac cccacagcct aggccgattc gacctctctc cgctggggcc     60
   ctcgctggcg tccctgcacc ctgggagcgc gagcggcgcg cgggcgggga agcgcggccc    120
   agacccccgg gtccgcccgg agcagctgcg ctgtcggggc caggccgggc tcccagtgga    180
   ttcgcgggca cagacgccca ggaccgcgct ccccacgtgg cggagggact ggggacccgg    240
   gcacccgtcc tgccccttca ccttccagct ccgcctcctc cgcgcggacc ccgccccgtc    300
   ccgacccctc ccgggtcccc ggcccagccc cctccgggcc ctcccagccc ctccccttcc    360
   tttccgcggc cccgccctct cctcgcggcg cgagtttcag gcagcgctgc gtcctgctgc    420
   gcacgtggga agccctggcc ccggccaccc ccgcg                                455
<210> 5
   <211> 20
   <212> DNA
   <213> 293 cell
<400> 5
   acaccgggac tgaaaatgag 20
<210> 6
   <211> 21
   <212> DNA
   <213> 293 cell
<400> 6
   cacaggttta caccttatgg c 21
<210> 7
   <211> 20
   <212> DNA
   <213> 293 cell
<400> 7
   ctgacctcat ggaggcttgg 20
<210> 8
   <211> 21
   <212> DNA
   <213> 293 cell
<400> 8
   gcccacacat ttcagtacct c 21